# EUROPEAN PATENT APPLICATION

(11) **EP 2 263 556 A1**
(43) Date of publication of application: **22.12.2010**
(21) Application number: 10184759.8
(22) Date of filing: 13.05.2005
(51) Int. Cl.: A61B 17/00

(54) **Percutaneous vein harvester**

(30) Priority: 13.05.2004 US 571598 P
(62) Divisional of application: 05749448.6
(71) Applicant: Medtronic, Inc., Minneapolis MN 55432-5604 (US)
(72) Inventor: Opie, John C., Scottsdale, AZ 85258 (US)
(74) Representative: Lawrence, John

(57) **Abstract**

A percutaneous harvesting device for the harvesting of tubular body members from a body is disclosed. The percutaneous harvesting device includes an endovascular guide for sufficiently straightening the tubular vessel to be removed and a perivascular cutting tool that is inserted over the endovascular guide. The cutting tool is advanced along the length of the tubular body member to be removed and it cuts body tissue (wherein the tubular body member is positioned inside the body tissue) at it is advanced. The body tissue is thus dissected from the body and can then be extracted percutaneously and rapidly from the body by pulling the endovascular guide, and the tubular body member and surrounding body tissue, from the body.

To be accompanied, when published, by Figure 13 of the drawings.

## Description

### CROSS REFERENCE TO RELATED APPLICATION

This application claims priority to U.S. Provisional Patent Application Serial No. 60/571,598 filed May 13, 2004 the contents of which are incorporated herein by reference.

### Field of the Intention

The present invention relates to methods and devices for removing tubular body members, particularly blood vessels, from the body of a human or animal.

### Background of the Invention

Various tubular structures in the body ("tubular body members") are sometimes removed, either for use somewhere else in the body or simply because removal is desired or necessary. As used herein, the terms "harvest," "dissect" and "remove," when used in connection with the removal of a tubular body member from the body, are synonymous. Tubular body members include blood vessels, such as arteries and veins, tendons, bile ducts and other structures. For example, the long sapheneous vein (LSV) located in subcutaneous fatty tissue in an anteromedial compartment of the lower leg and thigh is sometimes removed for use in (1) arterial bypass surgery, including coronary artery bypass, and peripheral arterial surgery bypasses, and (2) preparing an arteriovenus (AV) loop for dialysis. The length of the harvested LSV may vary from, for example, 20 cm to 100 cm.

Traditionally, the LSV has been removed by making a long incision along the leg from about the ankle to the groin, or by making a series of multiple, bridged incisions. Tissue (primarily fat) including the LSV is dissected from the leg through the incision(s) and the LSV is then dissecting from the surrounding tissue. While this procedure usually yields a usable LSV, the incision(s) has been painful, is reported to suffer wound healing failures rates of up to 40% (not infrequently mandating rehopsitalization and considerable expense and discomfort), is a possible source of infection, takes a long time to heal, and leaves a long, noticeable scar. Further, the harvested vein must be extensively handled in order to remove the surrounding tissue. That can result in damage to the LSV and possible early failure after the LSV is used as a graft.

In an attempt to solve the problems caused by removal of the LSV via a long incision or multiple incisions in the leg, various endoscopic techniques have been developed. Such techniques can involve the insertion of an endoscopic camera into the leg near the LSV at the knee area. The area around the camera may be inflated with a gas such as carbon dioxide using a gas-release nozzle positioned in an endoscopic dissection tool inserted along with the endoscopic camera - the gas is usually injected through a separate device requiring a separate incision. Such as endoscopic dissection tool is used to dissect the fatty tissue around the LSV and vein branches by gently using the pointed tip of the endoscopic dissection tool, the carbon dioxide gas flow and additional endoscopic dissection tools. After separating the LSV and vein branches from the fatty tissue, the dissection tools are withdrawn and an endoscopic clipper is used to clip the various branches. Once that is completed a cutting tool (typically cauterizing scissors) is inserted through the endoscope. The cutting tool is manipulated to divide and cauterize the clipped branches of the vein. As used herein, the term "divide" when used in relation to a tubular body member means to cut entirely through the tubular body member.

After the LSV is dissected and the branches are clipped and divided as described above, incisions to expose the vein are made through the skin at the distal and proximal ends of the leg. The vein is ligated in continuity and then divided with a pair of scissors. The dissected LSV is then pulled out of the body.

While these endoscopic procedures reduce scarring, pain, wound-related complications and risk of infection as compared to the previously described open incision method, the endoscopic technique is both difficult to learn and to use. Such an endoscopic procedure can also damage the vein due to over manipulation and potential mishandling of the endoscopic tools. Additionally, the endoscopic equipment used in these procedures is expensive to buy and use.

### Summary of the Invention

A method according to the invention improves upon the prior art methods for removing a tubular body member from the body and generally comprises the steps of (1) creating openings in the body through which the tubular body member can be accessed at a proximal end and a distal end, (2) sufficiently straightening the tubular body member to utilize a cutting tool according to the invention, (3) using a cutting tool to dissect a section of body tissue (wherein the tubular body member is inside the dissected section) between the proximal end and the distal end, and (4) removing the dissected section of tissue including the tubular body member from the body. Once the body tissue is removed from the body, the tubular body member is dissected from the body tissue using any suitable method.

One device according to the invention is a percutaneous harvesting device (PHD) for the harvesting of tubular body members from a body. The PHD includes (1) an endovascular component (EVC) for passing inside of the tubular body member to be removed, and (2) a perivascular cutting tool (PVT) that is inserted over the EVC and is used to cut a length of body tissue that includes the tubular body member.

In one preferred embodiment, the EVC comprises a guide wire and an endovascular guide (EVG) surrounding the guide wire. The EVG is preferably a catheter made from a soft material (preferably plastic) suitable for passing through the selected tubular body member without damage to the intimal surface of the tubular body member. The EVG may have a tapered end, or nose, to assist in introducing it into the tubular body member and may include one or more structures, such as grooves or rings, for securing the tubular body member to the EVG. Alternatively, the tubular body member can be secured to a specially designed, nozzle nosed, torque device with an external structure (such as an annular ridge or chevron) on the torque device, preferably positioned at the base of the nozzle. The nozzle is designed to fit partially inside the lumen of the tubular body member and to retain the tubular body member, preferably by a suture at the external structure for ligating the tubular body member to the torque device. The torque device is then lightened onto the guide wire at the proximal end and the distal end, thus the tubular body member is firmly fixed to the guide wire via the torque device.

The PVT is preferably cylindrical and has a diameter (or width) greater than the diameter of the tubular body member. The PVT surrounds the tubular body member and cuts through the body tissue surrounding the tubular body member thus dissecting from the body an essentially cylindrical section of body tissue (mostly fat in the case of the LSV) with the tubular body member inside the body tissue. If the tubular body member is a blood vessel, the blade cuts the branches of the blood vessel as it moves through the body tissue, thus isolating the blood vessel and enabling it to be removed without tearing. Once dissected, the dissected section of body tissue is removed from the body and the tubular body member is separated from the surrounding tissue in the dissected section.

In one preferred embodiment, the PVT includes a cutting head and a body section. The cutting head preferably has an annular leading edge that forms an angular cutting blade (although the cutting blade need not be annular or formed along the entire annular edge) and an inner cavity in which the tubular body member is received as it is dissected. The inner cavity of the cutting head may be funnel-shaped or have a funnel-shaped portion to assist in centering the cutting head and help avoid cutting or damaging the tubular body member. The cutting head may also include one or more exterior ridges that help guide the cutting head and, again, assist in keeping the cutting head straight so as to assist in preventing the tubular body member from being cut or damaged. The PVT optionally has one or more exit openings for extraneous body tissue received therein to exit, and such optional one or more openings could be on the cutting head.

The PVT may also be reticulated to move easily along the path of a tubular body member that is not straight. One way in which the PVT may be reticulated is by the cutting head being formed in a manner so that it can swivel.

Optionally, an inner sleeve may be positioned inside the cutting head. The purpose of the inner sleeve is to partially shield the cutting blade from the tissue, again to assist in preventing the tubular body member from being cut or damaged.

The body section of the PVT is preferably a plastic tube having an attachment structure (preferably threads) at one end for attaching to the cutting head. Optionally, the body section includes an outer surface (or exterior) having a helical thread or other device on the outer surface to assist in the movement of the cutting tool through the body. The body section also preferably has an inner cavity that communicates with the inner cavity of the cutting head. As the PVT moves and dissects the tubular body-member, the dissected tubular body member moves through the inner cavity of the cutting head into the inner cavity of the body section.

The PVT optionally is used in conjunction with a handle that can attach to an end of the body section of the PVT. The handle, and hence the PVT, is preferably turned by a user, such as a surgeon, to advance the PVT through a body to dissect the tubular body member. The handle may be an elongated shaft with one end that is connected to the body section of the PVT. Optionally, a handgrip can be attached to the handle for easier operation. Optionally, the PVT can be fitted with a power source, such as a battery pack and appropriate drive equipment to rotate or vibrate the PVT thus assisting in the dissection of the tubular body member with less resistance.

Thus, a PHD according to various aspects of the invention provides a less invasive and quicker way of removing tubular vessels such as the long sapheneous vein (LSV) from a body.

### Brief Description of the Drawings

Aspects of the present invention will be appreciated with reference to the description of the invention when made with reference to the accompanying drawings and wherein:

FIG. 1 is a side view of a preferred embodiment of a perivascular harvesting device;

FIG. 2 is a cross-sectional, side view of an embodiment of an endovascular component of the percutaneous harvesting device of FIG. 1;

FIG. 3 is a cross-sectional, side view of an alternate embodiment of an endovascular component;

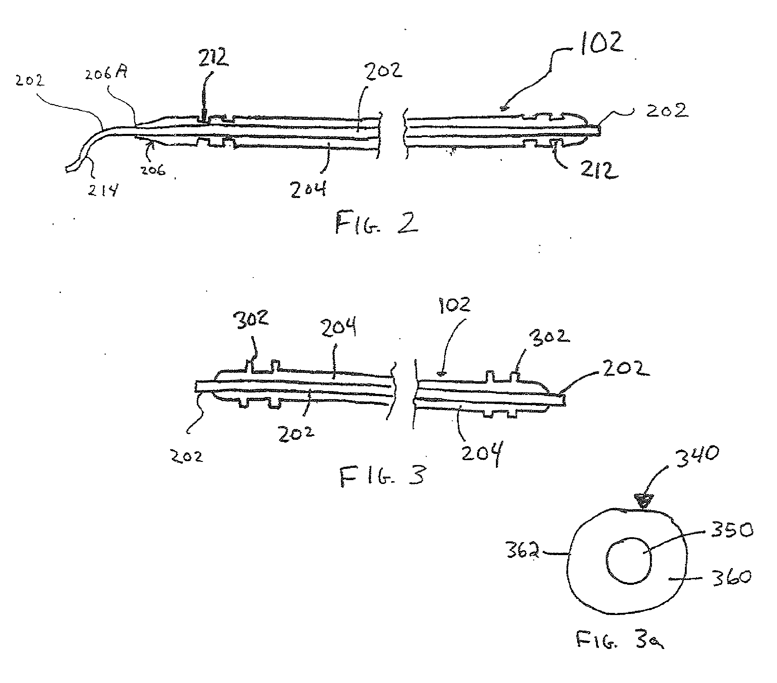

FIG. 3 a is an end view of an alternate embodiment of an endovascular component;

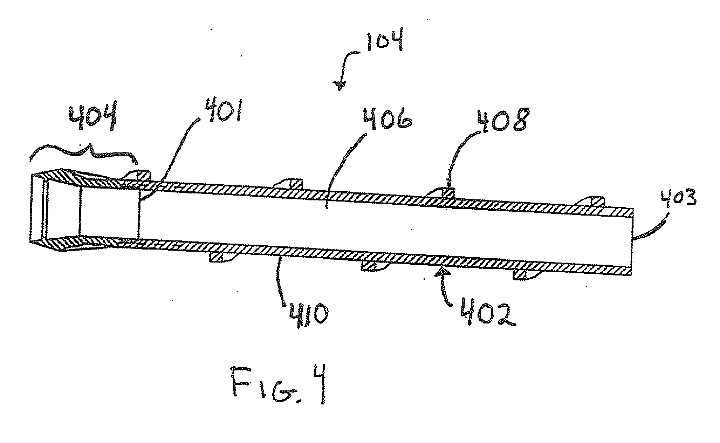

FIG. 4 is a cross sectional view of a perivascular cutting tool;

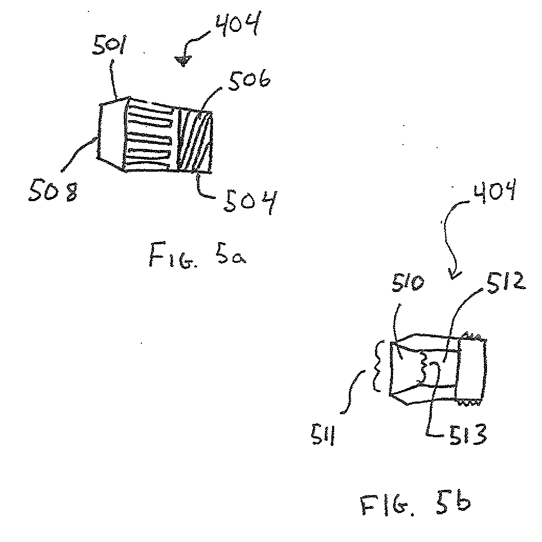

FIGS. 5a-5b are views of an embodiment of a cutting head for use with a perivascular cutting tool;

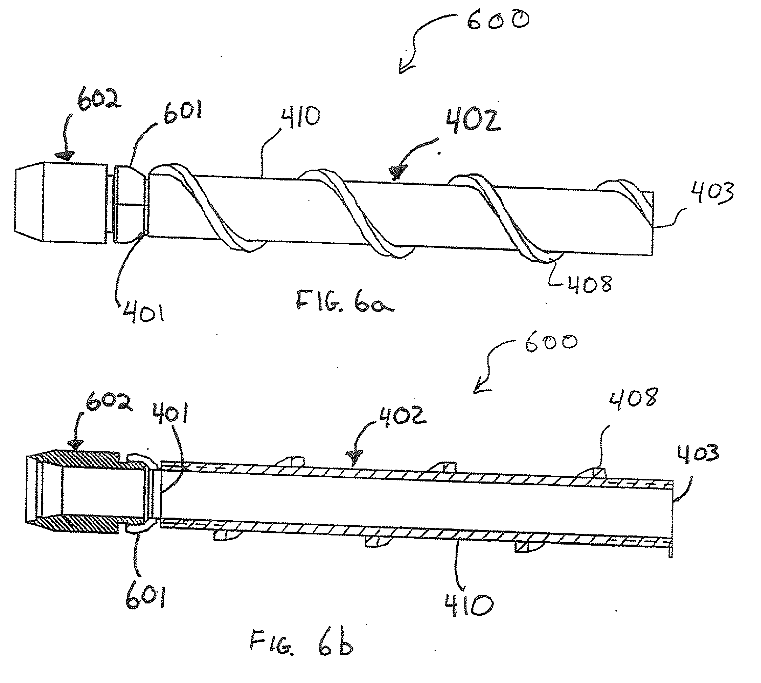

FIG. 6a is a side view of an alternative embodiment of a perivascular cutting tool;

FIG. 6b is a cross-sectional, side view of the perivascular cutting tool shown in FIG. 6a;

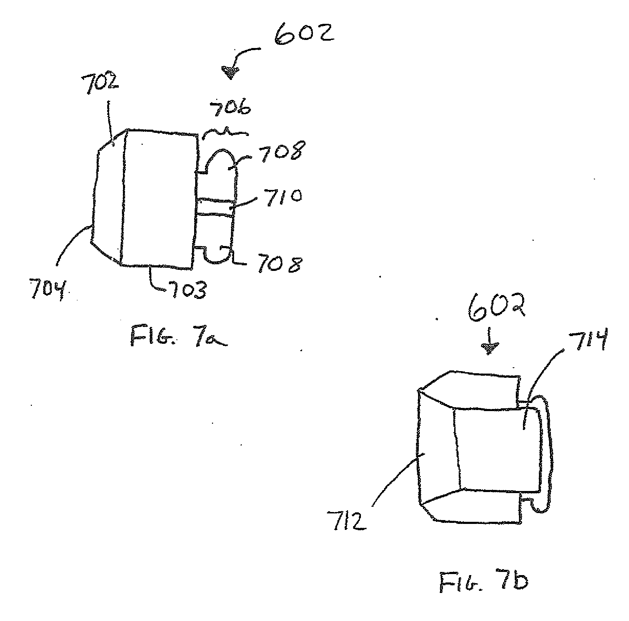

FIGS. 7a-7b are views of an alternative cutting head for use with the perivascular cutting tool of FIGS. 6a-6b;

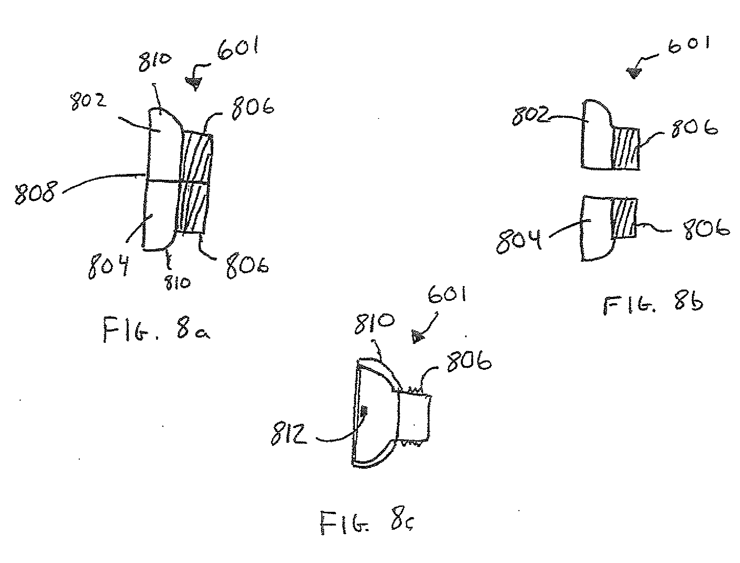

FIGS. 8a-8c are views of a connector for use with the cutting head of FIGS. 6a-6b and 7a-7b;

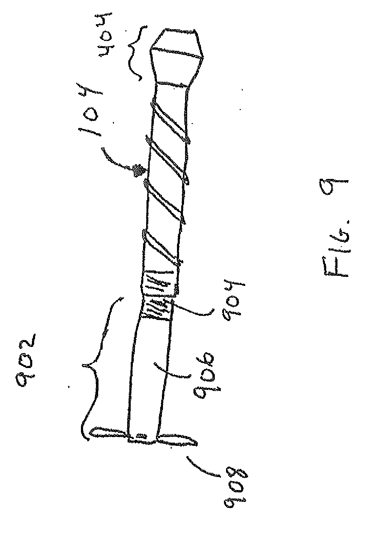

FIG. 9 is a view of a cutting tool according to the invention that includes an optional handle and an optional hand grip;

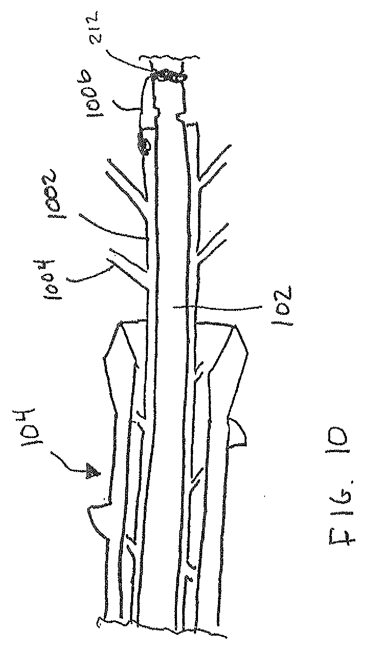

FIG. 10 is a view of a percutaneous harvesting device according to the invention in use;

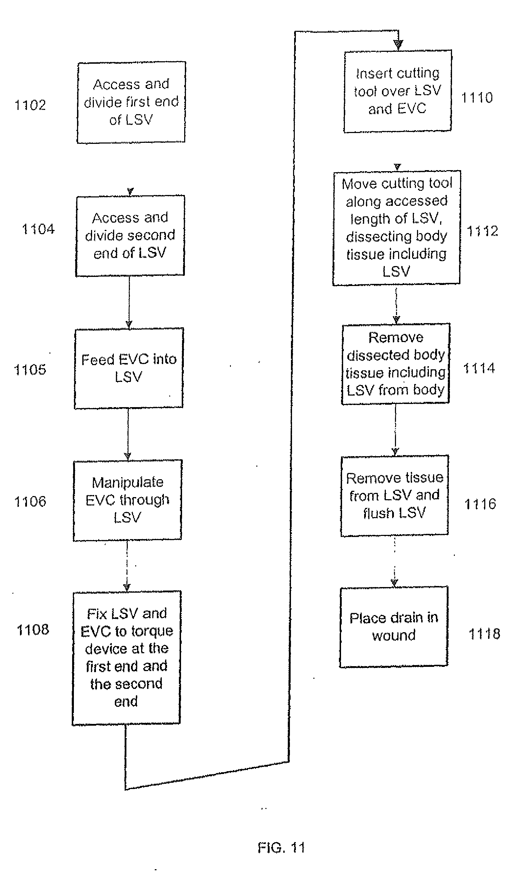

FIG. 11 is a flow diagram of a method using a device according to the invention.

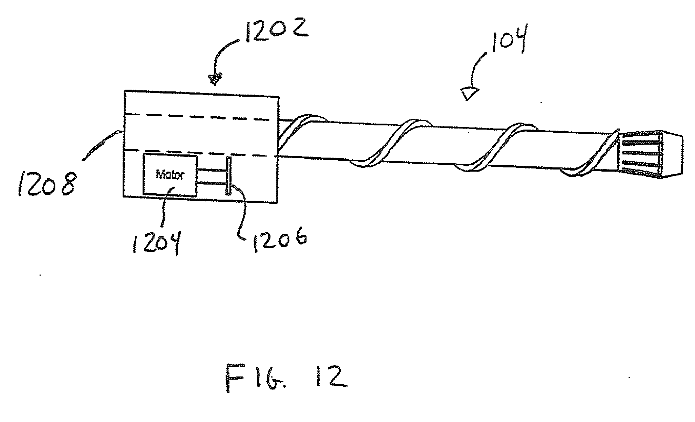

FIG. 12 is a view of an alternate embodiment of a perivascular cutting tool, which has an automatic advancement device.

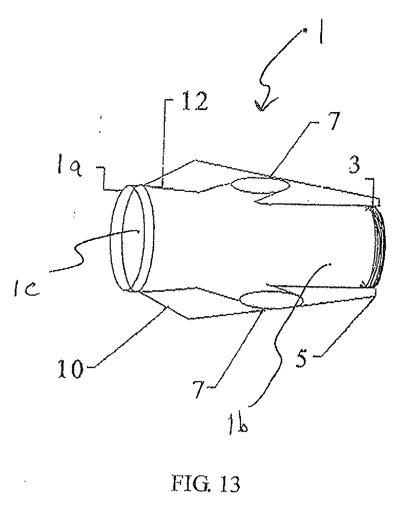

FIG. 13 is a side view of an alternative cutting head according to the invention.

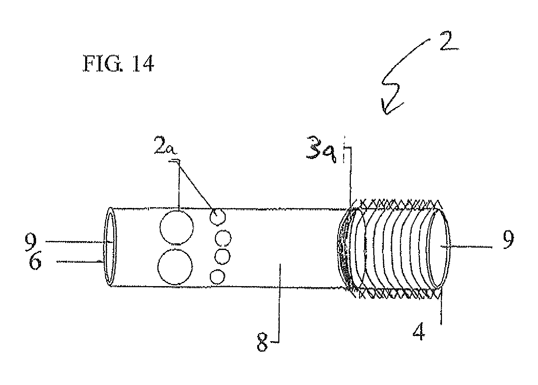

FIG. 14 is a side view of an optional sleeve that, if used, fits inside the cutting head of FIG. 13 blade.

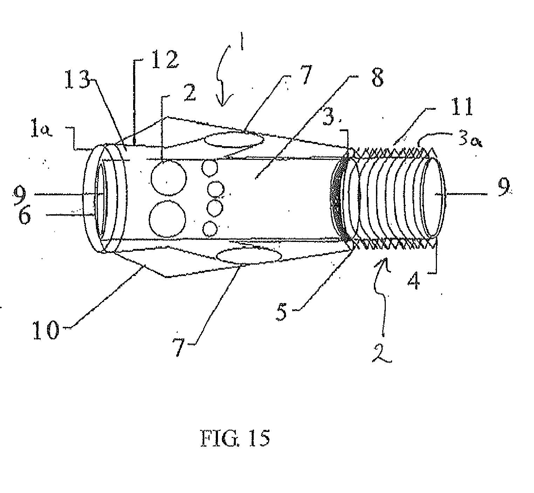

FIG. 15 shows the cutting head of FIG. 13 with the optional sleeve of FIG. 14 positioned therein.

### Detailed Description of the Preferred Embodiments

In the following descriptions, the present invention is frequently discussed using the example of the removal of a blood vessel, such as the LSV, from the body. However, the devices and methods of the invention can be used to harvest any tubular body member from a body for any purpose.

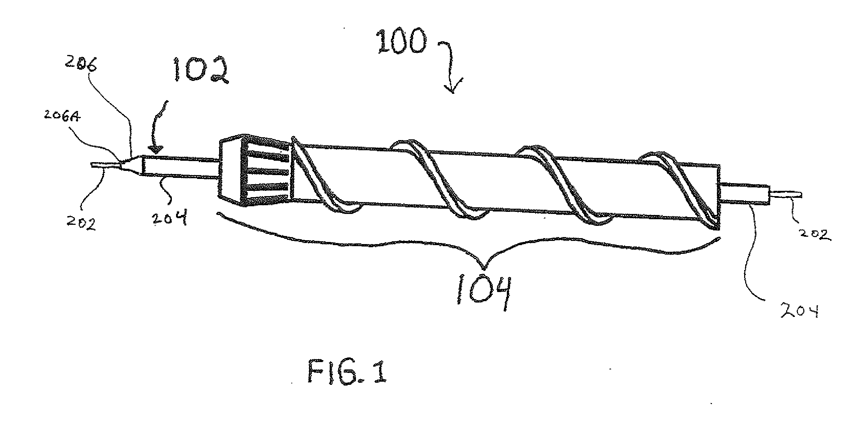

Turning now to the drawing figures where the purpose is to describe preferred embodiments of the invention and not to limit same, a preferred embodiment of one device of the invention is percutaneous harvesting device (PHD) 100, illustrated in FIG. 1. PHD 100 is used percutaneously for dissecting a tubular body structure, such as a blood vessel, by cutting through a length of body tissue that includes the tubular body member, thus freeing the body tissue including the tubular body member from the body.

PHD 100 includes an endovascular component (EVG) 102 for insertion into the blood vessel to be removed, and a perivascular cutting tool (PCT) 104 for traveling subcutaneously and coaxially outside of and along the length of the blood vessel in which EVC 102 has been inserted. Cutting tool 104 is for cutting tissue surrounding the blood vessel.

EVC 102 may be any structure or structures suitable for sufficiently straightening the blood vessel so that the blood vessel can be dissected from the body using a PVT according to the invention. Referring to FIG. 2, in one embodiment EVC 102 comprises a guide wire 202 and an outer tube, or endovascular guide (EVG), 204 surrounding guide wire 202, although it is possible that the EVC could be a single tubular member threaded through the vein. In the preferred method of using EVC 102, guide wire 202 is first inserted into the blood vessel and EVG 204 is then inserted over guide wire 202 and threaded through the blood vessel.

Guide wire 202 is any suitable medical guide wire that can be used in a procedure according to the invention, and guide wire 202 preferably has a hydrophilic coating and a straight floppy tip 214 to help provide maneuverability through the blood vessel. U.S. Provisional Application 60/475,666 to Opie and Joyce, filed on June 3, 2003 and entitled "Improved Medical Guide Wires" discusses exemplary guide wires and is herein incorporated in its entirety by reference. Suitable guide wires include those having a diameter between .010" - .038" and having about a 2-5 cm long floppy tip. The guide wire is long enough to pass through and extend outside of each divided end of the blood vessel and is preferably about 40% longer than the section of blood vessel to be removed. If used to remove an LSV, the guide wire is usually about 230 - 260 cm in length.

EVG 204 is preferably a flexible tubular plastic catheter and includes a central lumen 210 through which guide wire 202 is positioned when EVC 102 is positioned in the vein. EVG 204 optionally includes a tapered nose 206 that allows for easier introduction of EVG 204 into a blood vessel and easier passage through the blood vessel, and any structure suitable for this purpose may be used. Tapered nose 206 preferably is between about 3 and 4.5 cm in length and tapers to a tip 206A that is about 1.5 mm in diameter. EVG 204 can be soft enough to allow a suture to be secured to the EVG 204 in order to secure an end of a blood vessel thereto to facilitate removal. Alternatively the suture can be applied to a guide wire torque device to secure the blood vessel as described herein. A guide wire torque device (or simply "wire torque device" or "torque device") is a device that mechanically grips and secures a guide wire. Suitable wire torque devices are disclosed in copending application Serial No. 10/444,776 filed on May 24, 2003 and entitled "Guide Wire Torque Device" by Opie and Joyce, the disclosure of which is herein incorporated in its entirety by reference.

Typically, EVG 204 is 10% to 20% longer than the section of blood vessel to be removed because both ends of EVG 102 need to be exposed outside of the respective divided ends of the blood vessel, but EVG 204 may be of any suitable length. The outside diameter of the EVG depends on the size of the blood vessel because the EVG must be of a suitable size to pass through the blood vessel without damaging it. In a preferred embodiment, the EVG has a diameter of 3-4 mm.

In one embodiment, a series of optional grooves 212 are formed around the circumference of each end of EVC 102. Grooves 212 are for securing the blood vessel to EVC 102, preferably at each end of EVC 102 and preferably through the use of suture ligatures. In this embodiment the grooves are preferably about 0.5 mm deep and are spaced about 1.0 centimeter apart.

In another embodiment, grooves 212 (shown in FIG. 2) may be replaced by a series of optional rings 302 (shown in FIG. 2). In this embodiment, rings 302 are about 2.0 mm wide and are spaced about 1.0 cm apart. The blood vessel to be removed can be attached to rings 302 via sutures or clips. In addition to grooves 212 and rings 302, any other structure that allows a blood vessel to be attached to EVC 102 may be used, or EVC 102 may not include any such structure. Any such structures for attaching a blood vessel to EVC 102, if used, can be positioned at any suitable location on the EVC. In two known embodiments such structures are located on the EVG about 25-40 cm from each end of an EVG 80-150 cm in length, and 65-80 cm from each end of an EVG 230 cm in length. Alternatively, these structures may be replaced by structures on torque devices positioned at either end of the EVG, wherein the tubular body member can be secured to the structure on each torque device.

Presently, the most preferred embodiment of EVC 102 includes a guide wire with a hydrophilic coating and a single floppy tip and an EVG made of PVC or similar, suitable plastic, approximately 4 mm in diameter and having a central lumen of about 1-1.3 mm in diameter, wherein the EVG is threaded over the guide wire.

An alternate EVC 340 is shown in FIG. 3A. EVC 340 comprises an inner core 350 covered by an exterior covering 360. Inner core 350 provides both strength and flexibility to EVC 340, and is preferably made from a flexible or semi-flexible polymer plastic. However, any material that provides for a firm but semi-flexible inner core can be used. In one embodiment, the inner core is 2-3 mm in diameter. Any diameter can be used, however, as long as EVC 340 is properly sized to be threaded through the blood vessel to be removed.

The preferred embodiment of exterior covering 3 60 of EVC 340 is deformable and deforms in response to pressure from a suture or clip (such as a C-clip) in order to secure an end of a blood vessel to EVC 340 without significant slippage. The exterior covering can be made from foamed plastic, silastic or silicone rubber, although any suitable bio-compatible material can be used. In one embodiment, exterior covering 360 is 0.5 to 1.0 mm thick, although other thickness can be used with the maximum thickness controlled by the overall thickness of EVC 340, which needs to be properly sized to fit inside a blood vessel and is typically 3-4 mm in diameter if used in the removal of the LSV.

Exterior covering 360 may be co-extensive with inner core 350 or it may cover only part of an area of the inner core 350. In one embodiment exterior covering 360 may cover from 25 to 40 cm from one end of inner core 350 for a shorter EVC 340, or 65-80 cm from one end of inner core 350 for a longer EVC 340. EVC 340 may also include a nose or cone having dimensions the same as or similar to those of previously described structure 206.

The PHD further includes a perivascular cutting tool (PVT) 104. PVT 104 includes a body section 402 coupled to a cutting head 404. As used herein, unless otherwise stated, "coupled," "connected" or "attached" means attached in any manner suitable for the PVT to be used in the manner described herein.

Body section 402 is any suitable structure for use in a method according to the invention and is preferably a hollow tubular structure having a first end 401, a second end 403, a passage 406 extending therethrough and an optional driving helix 408 positioned on annular wall 410. Body section 402 is preferably an extruded, semi-flexible polycarbonate (such as General Electric Lexan 12) piece flexible enough to be suitable for the particular application in which it is to be used. Body section 402 supports cutting head 404 and preferably helps to substantially center the cutting head 404 around the blood vessel being removed during the cutting procedure. Body section 402, in one embodiment has an exterior diameter of 15 mm and an internal diameter of 10 mm and is approximately 100 cm in length, and in this embodiment wall 410 is preferably about 3 mm thick. Body section 402 may have different dimensions, however, the dimensions depending upon such factors as the application for which the PVT will be used and the amount of surrounding tissue to be removed with the blood vessel. The outer surface of wall 410 is preferably coated with a low-friction material, such as TEFLON, to reduce friction during use, or may be coated with a hydrophilic coating such as polyurethane.

Driving helix 408 is optional and is preferably a 2mm high, clockwise, helical thread positioned on (i.e., formed in or attached to) the outer surface of wall 410 of body section 402. Helix 408 assists in advancing PCT 104 through the body, and any structure positioned on wall 410 suitable for performing this function may be used, assuming such a structure is used at all. For example, other sizes and types of threads may be used, or a series of longitudinally-extending grooves may be positioned on in the outer surface of wall 410, and the grooves may be slightly twisted to provide gripping ability.

As PVT 104 is turned, driving helix 408 grips the body tissue through which it is passing and helps to advance PVT 104 forward, and/or helps to prevent PVT 104 from slipping backward during a procedure. In one embodiment, driving helix 408 is dimensioned such that for every 360° rotation of body portion 402, PVT 104 would advance 3.0 centimeters if there were no slippage, with a preferred range of 2-3 cm of advancement for every 360° rotation of body portion 402.

Cutting head 404 is designed to cut the body tissue surrounding the blood vessel and to cut blood vessel branches, thus dissecting the body tissue from the body so that the body tissue including the blood vessel can be removed. Cutting head 404 is preferably metal (most preferably carbon steel or stainless steel). Referring to FIGS. 5a and 5b, cutting head 404 has, in one embodiment, a generally wedge-shaped from 501 (as seen in side view), approximately in the shape of a truncated cone. The shape of front 501 assists in the movement of PVT 104 inside the body by pushing tissue outward from PVT 104 as cutting head 404 advances. While cutting head 404 with front 501 is illustrated, cutting head 404 can be any suitable shape for use on the PVT.

Cutting head 404 includes an attachment end 504. Attachment end 504, in one embodiment, has threads 506 that threadingly engage first end 401 of body portion 402, although cutting head 404 can be connected to body section 402 by any method or structure that provides a secure connection. Preferably, cutting head 404 is removable from body section 402, so that it may be disposed of (if desired), while body section 402 can be sterilized and reused (if desired).

Cutting head 404 includes a leading edge forming an annular blade 508. Alternatively, the cutting blade may not be annular or on the leading edge, although this is preferred, and the cutting blade may not be continuous. With annular blade 508 as shown at the leading edge of PVT 104 the force required to advance PVT 104 through the body tissue is less than the force that would be required if the blade was behind the leading edge. Further, the preferred annular blade provides 360 degrees of cutting surface, which also reduces the amount of force that must be applied to advance the PVT relative a cutting surface of less than 360°. Annular blade 508 is, in one embodiment, non-serrated, although a serrated annular blade can also be used.

Cutting head 404 also includes, in one embodiment, and with reference to the cross-sectional view of FIG. 5b, an internal funnel-shaped section or portion 510 coupled to internal cylindrical section 512. Internal funnel-shaped section 510 compresses tissue dissected by the cutting blade. In this respect, as PVT 104 advances, the dissected tissue is forced into section 510 by the forward movement of PVT 104. In section 510 the body tissue is compressed from a first diameter essentially equal to diameter 511, down to a second diameter essentially equal to diameter 513. The compression of the body tissue helps keep the PVT 104 essentially centered around the blood vessel to be removed, which helps to prevent the blood vessel from being cut or damaged by cutting blade 508.

In one embodiment first diameter 511, i.e., the diameter of annular blade 508, is 15mm and second diameter 513, i.e., the diameter of the internal cylindrical section 512, is 10mm. The length of internal funnel section 510 is, in one embodiment 10 mm, and is preferably in the range of 5 to 15 mm.

In another embodiment, and with reference to FIGS. 6a and 6b, an reticulated PVT 600 is shown. Reticulated PVT 600 has a reticulated (i.e., jointed) cutting head 602 that is able to move and pivot independent from body portion 402 and any structure suitable for allowing cutting head 602 to pivot may be used. The reticulation allows for easier movement of the reticulated PVT 600 around structures such as the knee. In one embodiment, reticulated cutting head 602 can pivot up to 15 degrees, although any suitable pivoting range may be utilized. Unless otherwise stated, the preferred size, shape, materials and configuration of cutting head 602 are the same as previously described for cutting head 404. Reticulated PVT 600 also includes a body section 402 (previously described) and a articulated connection section 601. Reticulated cutting head 602 is coupled to connection section 601, as best seen in FIG. 6b.

Referring to FIGS. 7a-7b, reticulated cutting head 602 includes a blade portion 702 having a leading edge forming an annular cutting blade 704 and a coupling section 706. Coupling section 706 includes an annular rim 708 and a channel 710 formed in rim 708. Similar to cutting head 404, reticulated cutting head 602 includes an internal funnel section 712 coupled to an internal cylindrical section 714. Internal funnel section 712 compresses tissue dissected by annular cutting blade 704. The advancement of reticulated PVT 600 forces tissue to the cylindrical section 714. The compression of the body tissue helps keep reticulated PVT 600 essentially centered around the blood vessel to be removed in order to assist in preventing the blood vessel from being cut by cutting blade 704.

Referring to FIGS. 8a-8c, preferred connection section 601 comprises two parts, a first connection section 802 and a second connection section 804. Each connection section 802 and 804 has threads 806 and when connection sections 802 and 804 are joined, threads 806 form an essentially continuous thread that can be used to threadingly connect section 601 to body portion 402. Connection sections 802 and 804, when joined, form a cup 808 between connection sections 802 and 804.

Cup 808 includes a lip 810 that engages and retains annular rim 708 of articulated cutting head 602 and enables cutting head 602 to pivot. Each lip 810 includes a stud 812. When cup section 808 is coupled to coupling section 706, each stud 812 is aligned with and positioned inside of a channel section 710. In one embodiment there are two channel sections 710, each of which has a stud 812 positioned therein when cutting head 602 is coupled to connecting section 601, This prevents the reticulated cutting head 602 from rotating continuously about a center axis, while still allowing the reticulated cutting head 602 to pivot freely. If the cutting head 602 were allowed to continuously rotate, then the twisting motion that may be used to advance reticulated PVT 600 inside the body could be translated into merely a spinning of reticulated cutting head 602.

Once the reticulated cutting head 602 is coupled to the articulated connection section 601, reticulated cutting head 602 and reticulated connection section 601 together act like a ball and socket joint wherein section 706 is analogous to the ball and cup section 808 is analogous to the socket. The reticulated assists in the advancement of PVT 104 through the body. For example, reticulated cutting head 602 allows PCT 600 to more easily maneuver around structures in the body, such as the knee joint.

Moving PVT 104 or 600 through the subcutaneous body tissue requires a certain amount of torque and/or pressure to force a PVT through the body tissue surrounding the blood vessel. To assist in advancing a PVT, an optional torque handle (or simply "handle") 902 can be attached to PCT 104 or 600 opposite the cutting head. Torque handle 902 is designed to fit into an adult human hand and is preferably a rod or tube made from extruded plastic (for example, Lexan 12, high density polyethylene, acetal, Nylon, ABS are all plastics that could be used) or any suitable material, and includes a connector 904 to connect to the PVT 104 and a shaft 906. In one embodiment connector 904 comprises threads on the rod or tube that threadingly connect to the PVT, Optionally a handgrip 908 can be attached to or formed in handle 902 to further assist in operation of the PVT.

In the preferred mode of operation, a surgeon or other medical worker would first attach torque handle 902 to the PVT 104 or 600. This is done using connector 904, which connects to the end of PVT 104 or 600 opposite the cutting head, preferably by a threaded connection, Once connected and the PVT is inserted into the body, the surgeon twists and pushes the shaft 906. This causes the PVT to rotate and move forward. Cutting head 404 or 602 cuts (i.e., dissects) the body tissue and the blood vessel and surrounding body tissue is pressed trough the cutting head 404 and body portion 402 as the PVT is advanced. Driving helix 408, if used, helps move the PVT forward and prevents the PVT from backing out of the body. To remove the PVT, the user would either advance it entirely out of the body or turn it in the opposite direction while pulling on the torque handle 902 to back it out of the body. Handgrip 908 can also be used to help in twisting the torque handle 902. Torque handle 902 and handgrip 908 could be made from a plastic such as polycarbonate, although any strong, rigid material can be used.

Referring now to FIG. 10, EVC 102 is shown positioned in a blood vessel 1002 to be removed. When EVC 102 is inserted into vessel 1002, blood vessel 1002 collapses around the endovascular guide 102 as the blood in blood vessel 1002 is pushed outward through branches 1004, As seen in FIG. 10, blood vessel 1002 can be secured to EVC 102 using a suture 1006 to secure to a structure 212 formed on EVG 204 or to the torque device as previously discussed. Alternatively, blood vessel 1002 may be secured to a wire torques device at one end or both ends. Typically, both ends of the blood vessel 1002 to be removed are secured to EVC 102 or to respective torque devices to help straighten blood vessel 1002 to be removed.

After endovascular guide 102 is inserted through blood vessel 1002, PVT 104 is passed along endovascular guide 102 such that endovascular guide 102 and blood vessel 1002 it is inserted into is inside PVT 104. As PVT 104 moves along endovascular guide 102 branches 1004 are severed by annular blade 508, which is on the leading edge of PVT 104. The diameter of annular blade 508 determines the length of branches 1004 left on removed blood vessel 1002. Cut blood vessel 102 and surrounding tissue passes into the inner channel of body section 402.

Referring to FIGS. 10 and 11, a preferred harvesting method shall be described. In this preferred harvesting (or removal) procedure, a PHD having either PVT 104 or PVT 600 may be utilized to remove an LSV. First, the LSV is accessed and divided at a proximal end (step 1102) and a distal end (step 1104). Next, a guide wire 202 is fed through the LSV and is exposed outside of the body at the proximal end and the distal end. EVG 204 is then advanced over guide wire 202 and into the LSV from the proximal end to the distal end and is exposed at each end (steps 1105 and 1106).

To secure the guide wire a wire torque device (the wire torque devices are not shown) is preferably placed on the guide wire at the proximal end outside of the LSV and another guide wire torque device is placed on the guide wire at the distal end outside of the LSV. The LSV is secured at both the proximal end and the distal end to either the EVG or a wire torque device, and the EVG is secured to a wire torque device at the proximal end and to a wire torque device at the distal end (step 1108).

The guide wire, catheter and LSV are then pulled straight by applying force to the distal end and the proximal end of each, preferably by pulling on the wire torque devices. As used throughout this application with respect to straightening a tubular body member, the word "straight" means sufficiently straight to utilize a cutting tool according to the invention, and is not limited to a perfectly straight configuration.

Once the LSV is sufficiently straightened to remove it using a PVT as described herein, a PVT is utilized to dissect body tissue including the LSV from the body. The PVT is positioned so that the guide wire and EVG are inside the PVT and the LSV is preferably approximately axially-aligned with the cavity of the cutting head. (Step 1110). Ideally, the passage of the PVT is coaxially aligned with the LSV, although the alignment need not be coaxial, the LSV must simply be positioned so that it is not cut by the cutting blade.

The PVT is then advanced along the accessed length of the tubular body member, cutting through the body tissue surrounding the LSV and the LSV branches, thereby separating the body tissue and LSV from the body (step 1112). Once separated, the tissue including the LSV is removed from the body, which may be accomplished by simply by withdrawing the EVG with the LSV and surrounding tissue out of one of the incisions (step 1114). After being removed, the LSV is dissected from the surrounding body tissue and the vein can be flushed and the branches tied off (step 1116).

A drain, optionally positioned in the PVT, can be placed into the wound created by the PVT. The PVT is then removed leaving the drain in the leg precisely where the body tissue had been (step 1118). The drain would then be in place to remove blood and clots from the wound. Exemplary drains are disclosed in U.S. Provisional Application 60/476,663 filed on June 5, 2003 and entitled "Improved Surgical Drains," to Opie and Joyce, the disclosure of which is herein incorporated in its entirety by reference.

In an alternative embodiment, manual operation of the PVT is replaced or augmented by an electro-mechanical operation using an automated device. For example, and with reference to FIG. 12, the perivascular cutting tool 104 is coupled to an automatic advancement device 1202. Automatic advancement device 1202 applies a twisting motion, vibration or other suitable force to the PVT to assist in advancing the PVT through the body and may be any device suitable for this purpose. In one embodiment, automatic advancement device 1202 comprises a low speed, high torque motor 1204 that couples to either the PVT or to a torque handle. Motor 1204 would use gears 1206, belts or any other method of connecting a motor to a shaft to transfer driving force to the PVT. Preferably, automatic advancement device 1202 comprises a variable speed motor to vary the torque an/or force applied to the PVT to control the speed of the PVT. In one embodiment, automatic advancement device 1202 includes an opening 1208 for the passage of an endovascular guide wire.

In addition to, or as an alternative to, twisting PVT 104 or 600, automatic advancement device 1202 may also vibrate or otherwise manipulate PVT 104 or 600 to assist in moving it through the body. Such a movement could be provided, for example, by an ultrasonic vibrator.

During cadaver trials utilizing reticulated cutting head 404 with an experienced operator it is usually possible to extract either an AK or a BK section of an LSV without observable damage to the LSV. Histology of these cadaver LSVs has disclosed no external media or adventitial or internal endothelial intimal damage. However, occasionally the cutting blade has injured an LSV. While large sections of the LSV would have been usable, some damage occurred because the blade on the reticulated cutting head wobbled and cut into the LSV. In thirty-one harvests, three LSVs were damaged. Thus the cutting head of one or more of Figures 13 and 15 was invented to help alleviate this problem.

Turning now to FIG. 13, a cutting head 1 is shown. Cutting head 1 is designed to help prevent a tubular body member being dissected from being cut or damaged by the cutting blade. Cutting head 1 may have the same dimensions and structures as previously-described cutting head 404 except for the differences shown in Figs. 13-15. Cutting head 1 as shown has a cutting blade 1a, a generally cylindrical body member 1b, defines an inner cavity 1c, and has an edge 5 opposite blade 1a. Cutting head 1 may be of any suitable shape, size and made of any suitable material, although it preferably is made of steel. Inner cavity 1c preferably has a funnel-shaped portion having the same dimensions as previously-described portion 510. Cutting head 1 optionally has one or more deflector ridges 10. The purpose of each ridge 10 is to deflect cutting head 1 away from hard or solid masses (such as bone) in the body in order to maintain the PVT on a proper path and help prevent the tubular body member being dissected from being cut or damaged by blade 1a.

Cutting head I may have one or more external vent channels, or exit openings, 7 to allow cut tissue fragments that enter cutting head 1 to be expelled through openings 7, thus reducing any tendency to clog or jam the PVT during use. Cutting head 1 may have optional internal threads 3 that lie within cavity 1c, wherein threads 3 are for connecting to sleeve 2, as described below. Cutting head 1 may also connect directly to a body section, such as body section 402, in any suitable manner. Cutting head 1 may have a sloping, cone-shaped outer body section 12, beginning at the distal end and sloping downwards towards edge 5, and cutting head 1 may have an exterior surface having the same shape as cutting head 404.

FIG. 14 shows a plastic or metal sleeve 2 that is dimensioned to be received in cavity 1c, and can be press fitted or screwed therein or received therein in any suitable manner. Sleeve 2 may have a rounded, or chamfered, leading (distal) edge 6 and sleeve 2 defines a lumen, or cavity, 9.

If sleeve 2 is used, it is positioned inside cavity 1c of cutting head 1 (shown in Fig. 15) so that leading edge 6 of sleeve 2 is set either at or just in front of blade 1a of cutting head 1 in a manner sufficient to help prevent blade 1a from coming in contact with the tubular body member to be dissected. Sleeve 2 may have one or more vent holes, or exit openings, 2a. Openings 2a are designed to allow cut tissue that could accumulate in space 13, shown in Fig 15, between blade 1a and sleeve 2 to be expelled. In this embodiment, behind one or more openings 2a is a non-vented section of sleeve 2. Non-vented section 8 of sleeve 2 terminates in an externally threaded section 3 a. Section 3a is designed to threadingly engage with the threads 3 of cutting head 1, as generally shown in Fig 15. Immediately following threaded section 3a is a second threaded section 11, which terminates at end 4. Threaded section 11 is designed to accept a matched threaded portion on the body section, although any suitable method of attachment may be used. For example, connection to a body section could be made via a pressure or snap fit, or the cutting head and body section could be integrally formed.

FIG. 15 shows a side view of the assembled cutting head 1 and sleeve 2. A slot 13 is formed between sleeve 2 and the sloping section 12 of cutting head 1. It: is noted the leading edge 6 of sleeve 2 is set at or just in front of cutting blade 1a, thus helping to prevent contact between blade 1a and the tubular body member to be dissected.

Due to the position of sleeve 2 and the fact that the tubular body member has been pulled straight, sleeve 2 will help to prevent blade 1a from cutting or damaging the tubular body member since sleeve 2 creates a space between the body tissue being cut and blade 1a. The thickness of sleeve 2 is a consideration. If sleeve 2 is too thick it could act as a resistor to blade advancement and advancement of the PVT, whereas if sleeve 2 is too thin it might tend to act as a blade itself. The leading edge of sleeve 2 could optionally be rounded to prevent it from cutting body tissue.

The operation of cutting head 1 could be, and is preferably, the same as for the previously described PVT utilizing cutting head 404.

Having now described preferred embodiments of the invention, modifications and variations that do not depart from the spirit of the present invention may be made. The invention is thus not limited to the preferred embodiments, but is instead set forth in the following claims and legal equivalents thereof.

### OTHER EMBODIMENT ARE SET OUT IN THE FOLLOWING CLAUSES.

1. A device for removing a tubular body member from a body, the device comprising a cutting tool that includes:
   (a) a cutting head having (i) a leading edge comprising an annular cutting blade, and (ii) an attachment section; and
   (b) a body section having a proximal end, a distal end and an inner passage extending therethrough, the distal end operable to couple to the attachment section of the cutting head;
2. The device of clause 1 wherein the cutting head has an exterior surface and one or more deflection ridges on the exterior surface, wherein the one or more deflection ridges assists in keeping the cutting head straight so as to help prevent the tubular body member from being cut.
3. The device of clause 1 wherein the cutting head further includes an inner cavity, at least a portion of the inner cavity being funnel-shaped and having a first inner diameter at the leading edge and a second inner diameter, the second inner diameter being smaller than the first inner diameter, the funnel-shaped portion of the inner cavity compressing body tissue during operation of the cutting tool to assist in keeping the tubular body member from being cut by the annular cutting blade.
4. The device of clause 3 wherein only a portion of the inner cavity is funnel shaped.
5. The device of clause 1 wherein the attachment section of the cutting head is threaded and the distal end of the body section is threaded, the cutting head attachable to the body section by threading it onto the distal end of the body section.
6. The device of clause 1 wherein the cutting tool includes one or more openings through which bodily debris can exit the tool.
7. The device of clause 6 wherein the one or more openings are on the cutting head.
8. The device of clause 1 wherein the cutting head has a longitudinal axis, and the device further includes a sheath that fits inside the cutting head, the sheath moveable along the longitudinal axis towards and away from the leading edge, the sheath for creating a space between the cutting blade and the body tissue so as to help prevent the tubular body member from being cut or damaged.
9. The device of clause 8 wherein the sheath has one or more vent openings through which bodily tissue can pass.
10. The device of clause 1 wherein the cutting tool is reticulated.
11. The device of clause 10 wherein the cutting head is reticulated.
12. The device of clause 10 wherein the cutting blade of the cutting head can tilt at least 15° in any direction.
13. The device of clause 10 wherein the body section further comprises an exterior surface, and a structure positioned on the exterior surface to assist in the movement of the cutting tool through body tissue.
14. The device of clause wherein the structure on the exterior surface is a helical thread.
15. The device of clause 10 further comprising an endovascular component for being positioned in the tubular body member.
16. The device of clause 15 wherein the endovascular component comprises a flexible tube and a medical guide wire.
17. The device of clause 15 wherein the endovascular component includes one or more structures to which the tubular body member can be attached.
18. The device of clause 10 wherein the cutting head is comprised of steel.
19. The device of clause 10 wherein the body section is comprised of polycarbonate.
20. The device of clause 10 that further comprises a handle attached to the proximal end of the body section.
21. The device of clause 19 wherein the handle comprises a cylindrical tube.
22. The device of clause 19 that further comprises a hand grip attached to the handle.
23. The device of clause 1 further comprising an automatic advancement device to assist in the movement of the device through the body.
24. A method for removing a tubular body member from a body, the method comprising the steps of:
   making a first incision;
   accessing and dividing a first end of the tubular body member near the first incision;
   making a second incision;
   accessing and dividing a second end of the tubular body member near the second incision;
   inserting an endovascular component into the first end of the tubular body member;
   moving the endovascular component through the tubular body member and out the second end so that the endovascular component has a proximal end exposed at the first end of the tubular body structure and a distal end exposed at the second end of the tubular body structure;
   securing the proximal end of the endovascular component and the distal end of the endovascular component and straightening the tubular body member by applying force to each end of the endovascular component;
   positioning a reticulated cutting tool having a leading edge comprising a cutting blade and an inner cavity extending therethrough so that the endovascular component is inside the inner cavity;
   advancing the cutting tool from the first end to the second end, the cutting tool cutting through and dissecting body tissue as it moves, wherein the tubular body portion is positioned within the dissected body tissue; and
   removing the dissected body tissue including the tubular body member.
25. The method of clause 23 wherein the cutting tool is advanced from the first end to the second end by utilizing a twisting or vibrating motion.
26. The method of clause 23 wherein the cutting tool is advanced by utilizing a motor that causes the cutting tool to twist or vibrate.
27. The method of clause 23 wherein the endovascular component is selected from the group consisting of a guide wire, catheter or balloon.
28. A reticulated cutting head used with a cutting tool for removing a tubular body member from a body, the cutting head comprising;
   (a) a leading edge comprising a cutting blade;
   (b) an inner cavity extending therethrough, the inner cavity comprising a funnel-shaped portion having a first diameter juxtaposed the leading edge and a second diameter, the second diameter being smaller than the first diameter; and
   (c) an attachment structure for attaching to a body section.

## Claims

1. An articulated cutting head, having a longitudinal axis, and used with a cutting tool for removing a tubular body member from a body, the cutting head comprising:
(a) a leading edge comprising a cutting blade;
(b) an inner cavity extending therethrough, the inner cavity comprising a funnel-shaped portion having a first diameter juxtaposed with the leading edge and a second diameter, the second diameter being smaller than the first diameter;
(c) an attachment structure for attaching to a body section; and
(d) a sleeve that fits inside the cutting head, the sleeve being moveable along the longitudinal axis towards and away from the leading edge, the sleeve designed for the purpose of preventing the blade from coming in contact with the tubular body member being dissected so as to help prevent the tubular body member from being cut or damaged.

2. The articulated cutting head of claim 1, wherein the cutting blade of the cutting head can tilt at least 15 degrees in any direction.

3. The articulated cutting head of claim 1 or 2, wherein only a portion of the inner cavity is funnel-shaped.

4. The articulated cutting head of any of claims 1 through 3, wherein the cutting head has an exterior surface and one or more deflection ridges on the exterior surface, for the purpose of deflecting the cutting head away from hard or solid masses in the body and helping prevent the tubular body member being dissected from being cut or damaged by the blade.

5. The articulated cutting head of any of claims 1 through 4, wherein the cutting head comprises one or more external vent channels through which bodily debris can exit the cutting head.

6. The articulated cutting head of any of claims 1 through 5, wherein the sleeve comprises one or more vent holes that allow for the expulsion of tissue that may accumulate in the space between the blade and sleeve.

7. The articulated cutting head of any of claims 1 through 6, wherein the cutting head further comprises an internal threaded section on the end opposite the blade and the sleeve further comprises an external threaded section, and wherein the threaded sections are designed to engage one another, attaching the sleeve to the cutting head.

8. The articulated cutting head of any of claims 1 through 7, wherein the cutting head further comprises an external threaded section on the end opposite the blade designed to accept a matched threaded portion of a cutting tool and attach the cutting head to a cutting tool.

9. The articulated cutting head of any of claims 1 through 8, wherein the cutting head is comprised of steel.
